# EUROPEAN PATENT SPECIFICATION

(11) **EP 0 487 750 B1**
(45) Date of publication and mention of the grant of the patent: **15.01.1997**
(21) Application number: 91911184.9
(22) Date of filing: 14.06.1991
(51) Int. Cl.: A61L 11/00

(54) **METHOD FOR MAKING AGGREGATE FOR CONSTRUCTION MATERIAL FROM INFECTIOUS MEDICAL WASTES**
VERFAHREN ZUR HERSTELLUNG VON AGGREGATEN FÜR BAUMATERIAL AUS INFEKTIÖSEN MEDIZINISCHEN ABFÄLLEN
METHODE POUR PRODUIRE DES AGREGATS POUR MATERIAUX DE CONSTRUCTION A PARTIR DE DECHETS MEDICAUX INFECTIEUX

(30) Priority: 18.06.1990 JP 157636/90
(43) Date of publication of application: 03.06.1992
(73) Proprietor: KANEKO, Tadashi, Yokohama-city, Kanagawa 246 (JP)
(72) Inventor: KANEKO, Tadashi, Yokohama-city, Kanagawa 246 (JP)
(74) Representative: Popp, Eugen, Dr.
(86) International application number: PCT/JP91/00801
(87) International publication number: WO 91/19518

(56) References cited:
- JP-A- 1 113 053
- JP-A- 1 115 359
- JP-A- 6 194 661
- JP-A-51 121 473

## Description

The present invention relates to a method for producing an aggregate employable for construction works from infectious medical waste articles derived from hospitals or the like by sterilizing them without any necessity for burning them.

As is well known, various kind of so-called infectious medical waste articles such as bloody injectors, injection needles or the like are discharged from hospitals as waste. Since there is a danger that infectious bacilli adhere to such infectious medical articles as mentioned above, disposal of the infectious medical articles in the same manner as ordinary waste is strictly inhibited. For this reason, personnels in a hospital collect infectious medical waste articles in a special container exclusively employable for them and then they dislocate the container to a disposing installation located outside of the hospital so that they are burnt and sterilized. However, since injections needles or the like remain in the disposing installation even after completion of the burning operation while their shape can visually be identified, infectious medical articles should be transported to a controllable type disposing installation for the purpose of thermal disposal at a higher cost than that in a case where ordinary waste is disposed in an ordinary disposing installation.

Since infectious bacilli can be sterilized at a temperature higher than 180 °C, this means that it suffices that infectious medical waste articles are heated up to a temperature higher than 180 °C without any necessity for burning them. However, due to the fact that various kinds of foreign materials, e.g., a synthetic resin such as polyvinyl chloride resin, polypropylene resin, acrylonitrile-butadiene-styrene copolymer resin or the like, a rubber and a metallic material are contained in the infectious medical waste articles, an unpleasant odor is generated from them as they are heated at a temperature higher than 160 °C. In addition, a toxic gas such as chlorine gas or the like is generated also from them as they are burnt. As far as a conventional method of sterilizing infectious medical waste articles merely by heating is concerned, an unpleasant odor and a toxic gas can not be processed at an acceptable cost. For this reason, they should unavoidably be disposed in the same manner as mentioned above by burning them in a combustion type disposing installation.

It should be added that disposal of infectious medical waste articles by an unauthorized operator is strictly prohibited, and moreover, a combustion cost and a transportation cost payable for disposing them are more expensive than those required for disposing ordinary waste, resulting in an economical burden to be borne by a hospital or the like being increased undesirably.

Since any proposal has been not hitherto made with respect to a method of reusing infectious medical waste articles, governmental organizations and local administrating organizations are required to yearly increase the number of controllable type disposing installations for disposing special waste articles. However, it is practically difficult to increase the number of disposing installations of the foregoing type and acquire a land or space for building the increased number of disposing installations.

The Japanese Patent Publication JP-A-1-115359 discloses a treatment method for reducing the volume of hospital refuse. The hospital waste is crushed by a rotary screw blade and subsequently heated to dry the resultant product. The dried waste is further crushed and mixed at high temperatures in order to melt the plastic waste component. The final product is a flake or rod-like material which is densely compressed.

The Japanese Patent Publication JP-A-1-113053 discloses a similar method for hospital waste disposal. The waste materials are sealed in a boiling vessel in the presence of water and are heated under pressure. Subsequently, the disinfected waste is fed to crushing means, whereafter the crushed material is classified into metal and other substances.

The present invention has been made in consideration of the foregoing background and its object resides in providing a method for producing a resuable product employable as an aggregate for construction works from infectious medical waste articles by heating them without any possibility that an unpleasant odor and a toxic gas are generated from them when they are thermally sterilized.

According to the present invention, there is provided a method for producing an aggregate material for construction works from infectious medical waste articles as defined in claim 1.

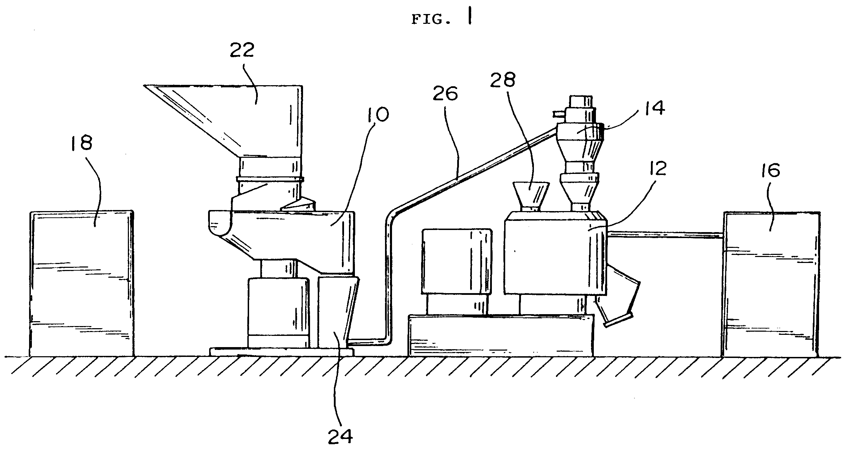

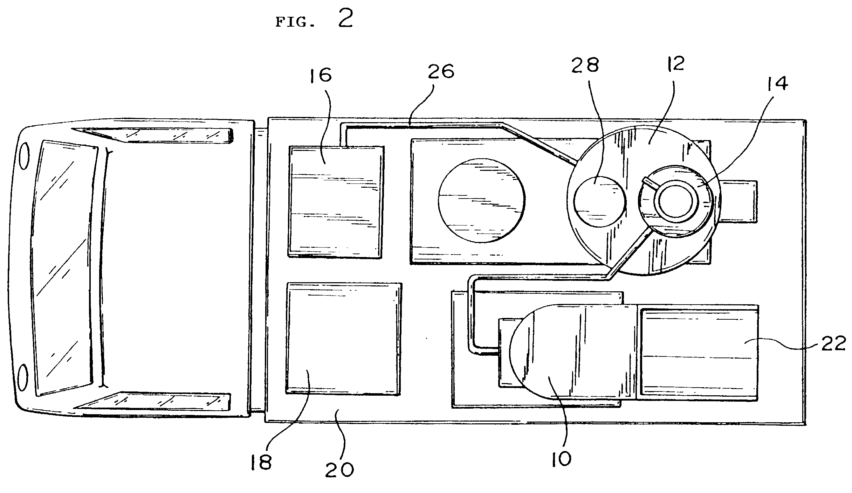

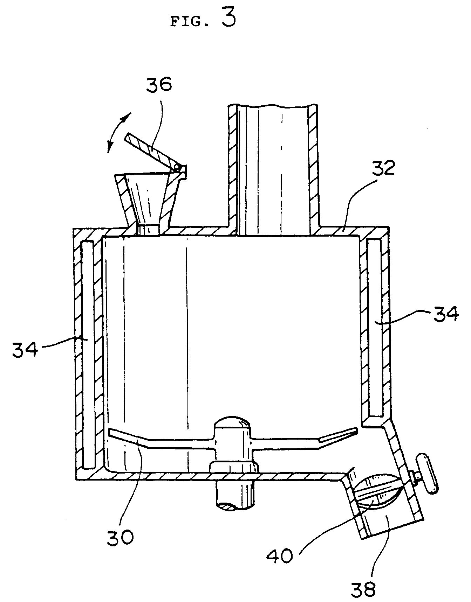

Fig. 1 is a side view which schematically illustrates in a disassembled state the structure of an apparatus for non-combustive sterilizing and reusing infectious medical waste articles. Fig. 2 is a plan view of the apparatus in Fig. 1, particularly illustrating an operative state wherein main components constituting the apparatus are mounted on a vehicle, and Fig. 3 is a sectional side view of a mixer constituting the apparatus in Fig. 1.

Fig. 1 is a side view which schematically illustrates in a disassembled state the structure of an apparatus for producing an aggregate employable for construction works from infectious medical waste articles in accordance with an embodiment of the present method. Fig. 2 is a plan view of the apparatus in Fig. 1 which illustrates an operative state wherein main components constituting the apparatus are mounted on a vehicle.

As is apparent from the drawings, the apparatus is essentially composed of a crusher 1 for crushing infectious medical waste articles into pieces, a mixer 12, a suction type supply unit 14 for introducing crushed waste articles into the mixer 12, an oil recirculation type heating unit 16 for heating the mixer 12, and a generator 18 serving as a power supply source for all the components as mentioned above. These components are mounted on an automotive vehicle 20 such as a truck as shown in Fig. 2.

As various kinds of infectious medical waste articles are supplied into the crusher 10 from a hopper 22 arranged above the crusher 10, they are crushed into pieces in the crusher 10 and the crushed pieces are stored in a storage chamber 24 located below the crusher 10. The reason why infectious medical waste articles are crushed into pieces in the crusher 10 is that injection needles or the like are crushed to form a safe material of which shape can not visually be identified. For this reason, the resultant product obtained by way of the foregoing method steps can be utilized as an aggregate for construction works. The suction type supply unit 14 arranged on the mixer 12 is communicated with the storage chamber 24 of the crusher 10 via a duct 26. As the suction type supply unit 14 is activated, the infectious medical waste articles which have been crushed in the crusher 10 are introduced into the mixer 12.

As shown in Fig. 1, a hopper 28 is mounted on the upper surface of the mixer 12 so that a pulverized catalyzer is supplied into the mixer 12 from the hopper 28. Referring to Fig. 3, the mixer 12 is equipped with a rotary blade 30 so that the crushed infectious medical waste articles and the pulverized catalyzer are vigorously stirred in the mixer 12 as the blade 30 is rotated therein. An oil recirculating passage 34 is formed around the side wall of a housing 32 of the mixer 12 so as to allow an oil to flow through the recirculating passage 34. As the oil heated in the heater 16 is recirculated through the recirculating passage 34, the interior of the mixer 12 is heated by the hot oil. At this time, the hopper 28 is covered with a lid 36, and an opening/closing valve 40 is disposed in an outlet port 38 of the mixer 12 located on the bottom of the same. While the blade 30 is rotated and the hot oil is recirculated through the recirculating passage 34, the lid 36 and the valve 40 should be kept closed.

Next, operation of the apparatus according to the present embodiment will be described.

First, infectious medical waste articles such as bloody injectors, injection needles or the like are introduced into the crusher 12. They are crushed into pieces in the crusher 10 to form a safe material of which shape can not visually be identified. This is intended so as to enable the crushed infectious medical articles to be utilized as an aggregate for construction works after they are sterilized.

Before the crushed infectious medical waste articles are introduced into the mixer 12, pulverized catalyzer is supplied into the mixer 12 from the hopper 28, and thereafter, the hopper 28 is airtightly closed with the lid 36. It should be noted that a catalytic material having two functions is selectively determined. One of the two functions is to assure that particles derived from the crushed infectious medical waste articles are not adhesively deposited on the inner wall of the mixer 12 and the other is to assure that an unpleasant odor generated from rubber based infectious medical waste articles at a temperature higher than 160 °C as well as a toxic gas such a chlorine gas or the like, generated when a polyvinyl chloride or similar resin is burnt, can completely be absorbed in the pulverized catalyzer. It has been found that a calcium carbonate is most preferably employed for the pulverized catalyzer. In addition to the aforementioned advantage, the calcium carbonate is available at an inexpensive cost, and moreover, can be used as a material of aggregate for elevating the strength of a concrete.

As hot oil heated by the heater 16 is introduced into the recirculating passage 34 arranged around the housing 32 of the mixer 12, the hot oil heats the mixer 12 to a temperature of about 150 °C so as not to allow unpleasant odor or toxic gas to be generated from the crushed infectious medical waste articles. After completion of the heating operation, the infectious medical waste articles crushed in the crusher 10 are conveyed to the mixer 12 via the duct 26 with the aid of the suction type supply unit 14. After a predetermined quantity of crushed infectious medical waste articles is introduced into the mixer 12, a communication port between the mixer 12 and the supply unit 14 is first closed. Subsequently, the blade 30 is rotated in the mixer 12. The crushed infectious medical waste articles and the pulverized catalyzer are mixed with each other in the mixer 12.

As the heated oil is recirculated through the recirculating passage 34, a mixture of the crushed infectious medical waste articles and the pulverized catalyzer is heated in the mixer 12 up to a temperature of about 150 °C. Additionally, frictional heat is generated as the blade 30 is rotated in the mixer 12, whereby a mixture of the crushed infectious medical waste articles and the pulverized catalyzer is heated to a temperature higher than 180 °C within a period of few minutes under the influence of the frictional heat. Once the crushed infectious medical waste articles have been heated to a temperature higher than 180 °C, infectious bacilli contained in the crushed infectious medical waste articles are completely sterilized. Although the mixture can be heated in excess of 180 °C by activating the heater 16, the temperature to be reached by the heater 16 is set to about 150 °C, since the temperature is elevated further by 30 °C or more merely by the frictional heat. When the temperature is raised up in excess of 160 °C, an unpleasant odor and a toxic gas are generated from the crushed infectious medical waste articles but they are absorbed in the pulverized catalyzer.

After the crushed infectious medical waste material have been completely sterilized, the valve 40 is opened. A mixture of the sterilized infectious medical waste articles and the pulverized catalyzer is discharged from the mixer 12 via the opening 38. The resultant product comprising the sterilized infectious medical waste articles and the pulverized catalyzer obtained via the opening 38 exhibits a ball-shaped mass having a diameter of 5 mm to 2 cm. It has been confirmed that the ball-shaped mixture can safely be used as an aggregate for concrete or similar construction material as it is, since the infectious medical waste articles have been crushed and completely sterilized.

The apparatus is brought in a hospital while the crusher 10, the mixer 12, the suction type supply unit 14 and the oil recirculation type heater 16 are mounted on an automotive vehicle 20 as shown in Fig. 3. Various kinds of infectious medical waste articles can be processed by an unskilled operator in the hospital without any necessity for particular skill by thermally sterilizing them in the apparatus. In this case, the crusher 10, the mixer 12, the supply unit 14 and the heater 16 are portably mounted on the automotive vehicle 20 but these components may be installed on the ground surface for practical use.

As described above, various kinds of crushed infectious medical waste articles are mixed with pulverized catalyzer in the mixer and the resultant mixture is then thermally sterilized by heating it in the mixture up to a temperature of 180 °C with the aid of frictional heat generated during a mixing operation as well as heating means. An unpleasant odor and a toxic gas generated from the crushed infectious medical waste articles during the heating operation at an elevated temperature are effectively absorbed in the pulverized catalyzer. In contrast with the conventional processing method as described above, there is no need of burning infectious medical waste articles for the purpose of sterilization. This leads to a very economical advantage for hospitals each of which has a task of disposing their infectious medical waste articles by itself.

In addition, a large amount of expenditure has been hitherto required for conveying infectious medical waste articles to a combustion installation or a controlled dumping location. In contrast with the conventional method, according to the present invention, infectious medical waste materials can economically be reused as an aggregate for construction works. Conventionally where infectious medical waste articles are disposed by conveying them away from a hospital, the disposing operation could be performed only by an well-experienced operator or an authorized operator. According to the present invention, however, each hospital can dispose waste by itself, and moreover, any company can perform disposing operations on a commercial basis so as to produce an aggregate employable for construction works from infectious medical waste articles.

Additionally, there is a possibility that government organizations or local administrating organizations may not be required to increase the number of control type dumping locations, when the the present method is employed for reusing infectious medical waste articles as an aggregate employable for construction works.

Since a calcium carbonate used as pulverized catalyzer is inexpensive, the resultant product, which has been subjected to sterilizing and deodorizing, offers a highly valuable product as an aggregate for concrete.

## Claims

1. A method for producing an aggregate material suitable for use in construction works from infectious medical waste articles, said method comprising the steps of:
a) crushing medical waste articles in a crusher (10),
b) mixing the crushed medical waste articles with a pulverized catalyzer in a mixer (12), the amount of catalyzer being effective for absorbing gas generated by the waste articles at elevated temperatures,
c) heating the mixture of step (b) by heating means (16) to thereby sterilize the mixture at a temperature higher than 180°C, the gas generated by the crushed medical waste articles being absorbed in the pulverized catalyzer, and
d) recovering the sterilized material after step (c) as said aggregate material.

2. The method of Claim 1, wherein said pulverized catalyzer is pulverized calcium carbonate.

## Patentansprüche

1. Verfahren zum Herstellen eines Zuschlagstoffs, der zur Verwendung bei Bauarbeiten geeignet ist, aus infektiösen medizinischen Abfallgegenständen, wobei das Verfahren die folgenden Schritte aufweist:
a) Zerkleinern von medizinischen Abfallgegenständen in einem Zerkleinerer (10),
b) Vermischen der zerkleinerten medizinischen Abfallgegenstände mit einem pulverisierten Katalysator in einem Mischer (12), wobei die Menge an Katalysator wirksam ist, um Gas zu absorbieren, das von den Abfallgegenständen bei erhöhten Temperaturen erzeugt wird,
c) Erwärmen des Gemischs von Schritt (b) durch eine Heizeinrichtung (16), um dadurch das Gemisch bei einer Temperatur von mehr als 180 °C zu sterilisieren, wobei das von den zerkleinerten medizinischen Abfallgegenständen erzeugte Gas in dem pulverisierten Katalysator absorbiert wird, und
d) Rückgewinnen des sterilisierten Materials nach Schritt (c) als den Zuschlagstoff.

2. Verfahren nach Anspruch 1, wobei der pulverisierte Katalysator pulverisiertes Calciumcarbonat ist.

## Revendications

1. Méthode de production d'agrégats utilisables dans des travaux de construction, à partir de déchets médicaux infectieux, la dite méthode comprenant les étapes de :
(a) broyage des déchets médicaux dans un broyeur (10),
(b) mélange des déchets médicaux broyés avec un catalyseur pulvérisé,dans un mélangeur (12), la quantité de catalyseur étant efficace pour absorber les gaz engendrés par les déchets à des températures élevées,
(c) chauffage du mélange de l'étape (b) par des moyens de chauffage (16) de façon à stériliser le mélange à une température supérieure à 180°C, le gaz engendré par les déchets médicaux broyés étant absorbé dans le catalyseur pulvérisé, et
(d) récupération de la matière stérilisée, après l'étape (c), sous forme de dits agrégats.

2. Méthode suivant la revendication 1, dans laquelle le dit catalyseur pulvérisé est du carbonate de calcium pulvérisé.
